# EUROPEAN PATENT APPLICATION

(11) **EP 2 345 897 A1**
(43) Date of publication of application: **20.07.2011**
(21) Application number: 10150988.3
(22) Date of filing: 18.01.2010
(51) Int. Cl.: G01N 33/92, G01N 33/68

(54) **Improved method for the detection of polyunsaturated fatty acids**

(71) Applicant: Nederlandse Organisatie voor Toegepast -Natuurwetenschappelijk Onderzoek TNO, 2628 VK Delft (NL)
(72) Inventor: Ramaker, Raymond, 8014 VZ Zwolle (NL); Verheij, Elwin Robbert, 3701 HW Zeist (NL); Coulier, Leon, 3994 NV Houten (NL); Tas, Albert, 4033 KE Lienden (NL); Bobeldijk-Pastorova, Ivana, 3991 JD Houten (NL)
(74) Representative: Hatzmann, Martin

(57) **Abstract**

The invention relates to a method to make a sample of fatty acids and bile acids suitable for analysis with liquid chromatography followed by mass spectrography (LC-MS) comprising derivatising said fatty acids and bile acids in the sample with glycidyltrimethylammonium chloride (GTMAC). The invention further pertains to a method for analyzing lipids, more especially free fatty acids comprising subjecting a sample to derivatisation with GTMAC and performing LC-MS on said sample. The invention further comprises the use of GTMAC for derivatisation of fatty and bile acids in an LC-MS analysis of a sample comprising said lipids

## Description

### FIELD OF THE INVENTION

The invention relates to the field of chemical detection technologies, more specifically to the field of detection of biochemical substances, more particularly polyunsaturated fatty acids, more particularly distinction of double bond fatty acid isomers in the presence of complex lipids.

### BACKGROUND OF THE INVENTION

Fatty acids play a role in the pathology of multiple diseases such as asthma, cystic fibrosis, type II diabetes, depression, epilepsy, inflammatory bowel diseases, atherosclerosis and some forms of cancer. Accordingly, methods of detecting these fatty acids in biological samples are of great interest for studying and monitoring these diseases. Especially the lipid composition in blood plasma can provide a wealth of information regarding the biochemical status of a subject and is important for diagnostic purposes. However, plasma, as most biological samples, is very complex in physicochemical terms because it is composed of a wide range of organic and inorganic constituents with a broad range of molecular weight and large differences in chemical structures. However, even in the case of closely related compounds, such as lipids and fatty acids, information is needed about the composition of the sample for specific compounds and a method that is suitable for the analysis of different compounds within such a related group is needed, as slight structural differences can account for large differences in the biological functions.

Fatty acids present in biological samples such as blood are present as free fatty acids (not bound in complex lipids or lipoproteins) or bound fatty acids. The latter can be analysed in the complex lipids or after alkaline hydrolysis.

The earliest attempts used gas chromatography (GC) to separate the group of fatty acids ( e.g. Schrade, W. et al., 1960, Klin. Wochenschr. 38, 126). These attempts were followed by the coupling of GC and mass spectrometry (MS) to provide structural data, such as the position of double bonds and heteroatoms (e.g. Murphy, R.C. et al., 2001, Chem. Rev. 101, 479). High performance liquid chromatography (HPLC) systems have also been used for the global analysis of fatty acid and complex lipid compositions. GC/MS and LC-UV analysis of fatty acids are roughly equivalent in terms of ease of use and sensitivity. To improve sensitivity in LC-UV methods derivation of the fatty acids is used, e.g. phenacyl derivatives (Borch, R.F. 1975, Anal. Chem. 47, 2437) or hydrazine derivatives (Miwa, H. et al., 1991, J. Chromatogr. 568, 25). Sensitivity is further increased in LC-MS studies by using atmospheric pressure chemical ionization (APCI), especially for the analysis of unsaturated fatty acids. Overall, LC-MS methodology is more sensitive than LC-UV methods.

Rezanka (2000, J. High Resolut. Chromatogr. 23, 338) showed that LC-APCI/MS allowed for the analysis of thermally unstable polysaturated fatty acids. LC-APCI/MS/MS analysis of pentafluorobenzyl (PFB) derivatives of fatty acids provided useful product ions that could be used for high sensitive applications (Singh, G. *et al.,* 2000, 72, 3007), especially for unsaturated fatty acids and their metabolites (Lee, S.H. et al., 2005, Prostaglandins Other Lipid Mediat. 77, 141). However, PFB derivatives are not useful for saturated fatty acids. Recently, Pettinella et al. (2007, J. Chromatogr. B, 850, 168) showed a sensitive system for analysis of both saturated and unsaturated fatty acids using derivatisation with trimethylaminoethyl (TMAE). There are not many examples in the literature where free fatty acids and complex lipids can be analysed within one analysis, therefore there is still need for further methods for the analysis of lipid and especially fatty acid compositions.

### SUMMARY OF THE INVENTION

The present invention now relates to a method to make a sample of fatty acids and bile acids suitable for analysis with liquid chromatography followed by mass spectrography (LC-MS) comprising derivatising said fatty acids and bile acids in the sample with glycidyltrimethylammonium chloride (GTMAC). Also a subject of the invention is a method for analyzing lipids comprising:
a. subjecting a sample comprising lipids and free fatty or bile acids to derivatisation with GTMAC; and
b. performing LC-MS on said derivatised fatty or bile acids in the presence of intact lipids.

In the above methods according to the invention the sample preferably comprises fatty acids, more particularly polyunsaturated fatty acids (PUFAs). Also preferably said sample is a biological sample, more preferably a blood, plasma or serum sample. Further preferred in a method according to the invention, the LC is ultra-high pressure liquid chromatography (UHPLC). Further, preferably the MS analysis is performed by electrospray (ESI). In another aspect of the invention preferably the derivatisation is performed at a temperature between 0°C and 90°C, preferably between room temperature and 80°C, more preferably around 65°C and the derivatisation reaction is performed for more than 30 minutes, preferably for more than 60 minutes, more preferably for 85 minutes or more, and most preferably between about 87 and about 93 minutes.

The invention also comprises the use of GTMAC for derivatisation of fatty and bile acids in an LC-MS analysis of a sample comprising said lipids.

### LEGENDS TO THE FIGURES

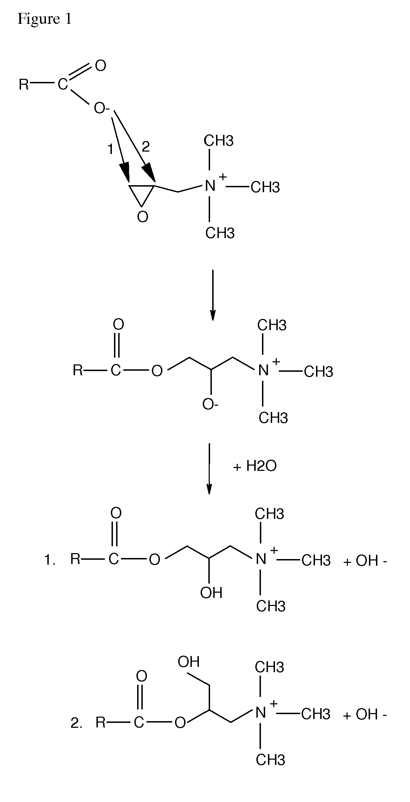

Figure 1. Reaction mechanism of the derivatisation reaction.
Figure 2. Sensitivity of the method shown as the signal (peak) intensity measured when injecting 2, 5 and 20 fmol of two isomers of C20:4 fatty acid on column.
Figure 3. Ion chromatogram showing separation of two positional isomers of derivatised C20:4 free fatty acid and in the same analysis the detection of an intact complex lipid, LPC. Analysis in standard and human plasma extract.
Figure 4. Detection of derivatised deuteradted and endogenous bile acids after derivatisation of a plasma extract with the described method.
Figure 5. Differences in fatty acid composition of one human subject within a clinical study where next to a control treatment also a fish oil supplementation was used. After fish oil supplementation, fatty acids present in high concentrations in fish oil are also detected in plasma in elevated concentrations.

### DETAILED DESCRIPTION

In the present method the derivatisation of the fatty acids and bile acids in the sample to be tested with GTMAC provides major advantages over the prior art of Pettinella et al., who used TMAE for the derivatisation of fatty acids which were analysed with LC-MS.

First of all, the above-mentioned prior art method is not suitable for the analysis of any sample containing free fatty acids. The method is applicable to the analysis of total fatty acids after alkaline hydrolysis of all lipids in the sample or to free fatty acids but after a fractionation step where all free fatty acids are separated from complex lipids prior to derivatisation. The method of the present invention is suitable for both derivatisation of free fatty acids in the presence of (complex) lipids without prior fractionation as well as derivatisation of total fatty acids after alkaline hydrolysis of the lipids.

Secondly, the prior art method with TMAE uses a pretreatment with oxalylchloride which is essential for the further derivatisation of the fatty acids with TMAE. In the method of the present invention such a pretreatment is not needed, which means that the GTMAC derivatisation can be performed directly on the sample.

Thirdly, during the oxalylchloride treatment that is necessary for TMAE derivatisation, hydrochloric acid can be produced which causes degradation of complex lipids that are present in the sample. By this degradation fatty acid composition in the sample changes. Furthermore, even in the absence of complex lipids (after hydrolysis or fractionation), hydrochloric acid can cause unsaturated fatty acids in the sample to isomerize, by which new or other fatty acids are formed, and which thereby influences the levels of fatty acids in the samples, and thus yields erroneous measurements.

Furthermore, TMAE derivatisation needs to be performed under water-free conditions. This necessitates elaborate drying of the sample (e.g. under nitrogen atmosphere). Derivatisation with GTMAC can take place under aqueous conditions, which means that the derivatisation can directly be performed on the (slightly) aqueous extract of the (biological) sample.

Lastly, derivatisation with TMAE is a two-steps reaction: first treatment with dimethylaminoethanol and after drying, further treatment with a methyl iodide/methanol mixture. Derivatisation with GTMAC is a simple, one-step reaction when total fatty acids are derivatised. When free fatty acids are derivatised in the presence of complex lipids, the reaction needs to be terminated by adjusting the pH.

Accordingly, the present invention is a major improvement over the currently used prior art method, while maintaining the excellent analytical properties of the prior art method. As will be shown in the experimental section, the present method is capable of giving a combination of chromatographic and MS results from which the individual fatty acid molecules, even the positional isomers of unsaturated fatty acids (PUFAs) and also bile acids and other lipids of interest can be recognized. Further, the method does not only provide a qualitative indication of the presence of one or more particular lipids in the sample, but it allows for a quantitative determination of the level of the lipid in the sample where standards are available.

The method of the invention can be used to determine a large number of fatty acid, bile acid and lipid molecules in a sample. The sample is preferably a biological sample, such as a blood plasma or serum sample or a sample made from a tissue homogenate, e.g. from liver or adipose tissue. For using a blood derived sample, the commonly used anticoagulant compounds, such as heparin, EDTA or coumarin may be used without disturbing the analysis.

The groups of lipids that can be analysed with the present method comprises free fatty acids including positional isomers of unsaturated fatty acids, bile acids, lysophosphatidyl cholines, phosphatidyl cholines, sphingolipids such as sphingomyelin, etc.

The preparation of the sample for use in the current invention depends on the nature of the sample: when the sample is a liquid, such as a sample from blood, serum or plasma, only the proteins are removed by precipitating with isopropanol. Because of this simple procedure, very low volumes of blood, serum or plasma can be processed, as low as 5 microliters. When the sample is a tissue sample, the sample should be homogenized, e.g. by ultrasonication. Further, the sample should be extracted with a suitable solvent and centrifuged to remove any solid debris from the sample.

In general, the sample can then be transferred to the vial that is used for the chromatography and the derivatisation reaction can be performed in said vial. Derivatisation is performed by adding an excess amount of GTMAC to the sample. The derivatisation reaction can be performed by any temperature between 0°C and 90°C, but, as will be shown in the Examples, the best results are obtained by temperatures between room temperature and 80°C, more preferably at a temperature of about 65°C. The sample is incubated with the reagent for at least 30 minutes for qualitative results, preferably for at least 60 minutes and for quantitative results the incubation time is at least 85 minutes. Preferably, the incubation time is not longer than 120 minutes, more preferably not longer than 100 minutes, and especially preferred is an incubation time between about 87 and about 93 minutes. This incubation time is preferred because it is substantially long to have the derivatisation reaction completed, while any longer reaction would also affect the fatty acids that are available in the complex lipids. As has been discussed above, freeing the fatty acids that are available in complex lipids would negatively influence the reliability of the assay. After the reaction period the reaction is terminated by adding termination reagent (a solution of formic acid to adjust the pH). Further, derivatisation is preferably conducted in a buffered sample with a pH above 7, preferably above 8 and more preferably of about pH 8.8.

Subsequent chromatography is then performed on the (liquid) sample. Preferably the chromatography is high pressure LC, more preferably ultra-high pressure LC. This chromatography can be performed with many eluents and column combinations, depending on the specific lipid groups that need to be analysed in addition to the free fatty acids. For example if the analysis is limited to free fatty acids and the more polar lipids such as lysophosphatidyl cholines, phosphatidylcholines and sphingomyelins preferable is a C8 column with methanol and acid ammonium salt solution. If the analysis mainly concerns (free) fatty acids, a C18 column or e phenyl column could advantageously be used. It is submitted that a person skilled in the art will be able to select the column type that is most appropriate for the sample that needs to be analyzed. A similar aspect concerns the eluents that are used to elute the fatty acids and/or lipids from the column. As will be seen in the experimental part, a gradient of different eluents is preferably used to be able to analyse as many fatty acids and/or lipids as possible. Also in this respect, the person skilled in the art will be able to select the eluents which are the most appropriate for the sample to be analysed.

The mass spectrometry can be performed by any means known in the prior art. Preferably, the MS is performed with electrospray ionization in the positive ion mode (ESI+). Because after derivatisation the (fatty) acids ionize very well, and even the positional isomers of the unsaturated fatty acids are baseline separated the detection can be performed in full scan, MS/MS detection is optional. Any type of conventional mass analysers can be used, such as triple quadrupole, ion trap, time-of-flight.

From the experimental section it will be clear that the above described method enables identification of the single (free) fatty acid components that are present in the sample. It is possible to correctly identify different isomers of fatty acids, such as the couples C18:3-. 3 and C18:3-. 6; C20:3-. 3 and C20:3-. 6; and C20:4-. 3 and C20:4-. 6. Also a good discrimination was found with relation to the bile acids cholic acid, lithocholic acid, deoxycholic acid and glycodeoxycholic acid.

### EXAMPLES

### Example 1

### Experimental

### Materials and reagents

Methanol (Chromasolve) was purchased from Riedel de Haen (Seelze, Germany), formic acid (p.a. grade) and hydrochloric acid , 37% was from Merck (Darmstadt, Germany). Isopropanol (IPA) was from JT Baker (Deventer, Netherlands) and acetonitrile was from Biosolve (Valkenswaard, Netherlands). Millipore ultra-pure water was used, prepared by the in-house purification system.

From Sigma Chemical (Steinheim, Germany) the following chemicals were purchased: Glycidyl Trimethyl Ammonium Chloride (GTMAC), TRIS- base and ammoniumformiate (NH₄COOH).

Fatty acid standards were purchased from Cayman Chemical Company (Michigan, USA) : myristic acid (C14:0-FA), palmitic acid (C16:0), palmitoleic acid (C16:1), stearic acid (C18:0), oleic acid (C18:1), linoleic acid (C18:2), alphalinolenic acid (C18:3-ω3), gamma-linolenic acid (C18:3-ω6), dihomo-gamma-linolenic acid (C20:3-ω6), mead acid (C20:3-ω9), eicosatetraenoic acid (C20:4-ω3), arachidonic acid (C20:4-ω6), docosapentaenoic acid (C22:5-ω3) and docosahexaenoic acid (C22:6-ω3).

Internal standards: Deuterated fatty acids were purchased from CDN Isotopes (Quebec, Canada) or from Sigma-Aldrich. D31-palmitic acid (C16:0-D31), D33-heptadecanoic acid (C17:0-D33), deoxycholic acid-d4, lithocholic acid-d4, cholic acid-d4 and glyco-lithocholic acid-d4 were used. For complex lipids, 1-a-heptadecanoyl-lysophosphatidylcholine (C17:0-LPC), 1-a-dilauroyl phosphatidylcholine (C24:0-PC) were purchased from Sigma and used as internal standards.

### Calibration and internal standard preparation

The stock solutions of the individual lipids were prepared separately in 10% dichloromethane in isopropanol (all lipids except for fatty acids) or in pure isopropanol (only fatty acids) and were stored at <-18 °C.

Calibration standards were prepared by combining appropriate volumes of each stock solution and isopropanol.

Calibration ranges for the individual lipids and fatty acids were estimated based on concentrations described in the literature or in the Human Metabolome database (www.hmdb.ca). Selected high abundant and low abundant lipids and fatty acids were included in the calibration curves. The calibration range for the polar lipid platform was from 5nM to 20uM in the final solution.

For extraction, internal standard solution in IPA (isopropanol) was prepared containing all IS (internal standards) in relevant concentrations (depending on the expected concentrations in the sample).

### Sample preparation, derivatization

For plasma and serum, sample aliquots (a minimum of 5 µ1) were deproteinized and extracted with 30 aliquots of IPA containing internal standards. After centrifugation, an aliquot of 50 µ1 of the clear supernatant was transferred to a new vial already containing containing the derivatisation reagent, GTMAC, 2.5 (volume) diluted with a 100 mM TRIS solution in Milli water adjusted to pH 7.2 with 1 M HC1 solution. The vials were capped and vortexed. The rack was placed into a stove and kept at 65°C +/- 2°C for 90 minutes. After 60 minutes the whole rack was vortexed briefly and placed back into the stove. After 90 minutes, the vials were opened, 150 µL of the termination solution was added (1% Formic acid solution in IPA). The samples were capped and vortexed and injected in the LC-MS device.

### LC-MS analysis

LC-MS analysis was carried out using a ThermoElectron LTQ-Orbitrap, equipped with a ThermoElectron Accela UHPLC with autosampler and column-oven (ThermoElectron, Breda, The Netherlands). The instrument was equipped with an ESI source operated in positive ion mode.

1 µL of the clear extract (maintained at 20°C in the autosampler) was injected onto a C8 column (Waters Acquity C8 1.7 µm particle size, 2.1mmx100mm). Compounds were separated at a flow-rate of 500 µL/min using a methanol-aqueous formic acid (FA) gradient. Mobile phase A (A) was water containing 0.1% FA, mobile phase B (B) was acetonitrile containing 0.1% FA, mobile phase C (C) was 2 mM NH₄COOH in methanol containing 0.1% FA, mobile phase D was 0.1% of FA in IPA. For needle wash, mobile phase C was used. The gradient is shown in Table 1.

**Table 1. Gradient for the separation of free fatty acids, bile acids and polar lipids**

| Time (min) | Flow (ml/min) | %A | %B | %C | %D |
|---|---|---|---|---|---|
| 0 | 0.5 | 98 | 2 | 0 | 0 |
| 0.5 | 0.5 | 98 | 2 | 0 | 0 |
| 0.6 | 0.5 | 60 | 40 | 0 | 0 |
| 8.5 | 0.5 | 35 | 65 | 0 | 0 |
| 9 | 0.5 | 0 | 0 | 100 | 0 |
| 12 | 0.5 | 0 | 0 | 100 | 0 |
| 12.1 | 0.5 | 0 | 60 | 0 | 40 |
| 15 | 0.5 | 0 | 60 | 0 | 40 |
| 15.1 | 0.5 | 98 | 2 | 0 | 0 |
| 18 | 0.5 | 98 | 2 | 0 | 0 |

The column was stabilized at the initial gradient for 3 min before injecting the next sample. The total separation time was 18 min including stabilization of the column. The column temperature was maintained at 50 °C. After separation, eluting compounds were ionized using ESI in the positive ion mode and detected in full scan, range m/z 202-1000.

Operating conditions of the MS were as follows: Spray voltage at 4 kV, and the heated capillary at a temperature of 300°C. Nitrogen was used as sheath, auxiliary and sweep gas, set at 35, 15 and 5 (in arbitrary units) respectively.

The capillary voltage was 10 V and the voltage of the Tube Lens was 60V. The detection was performed in the Orbitrap in full scan. Using accurate mass, the resolution was set at 7500 at m/z 400. The data were processed using the LC Quan software (ThermoElectron, Breda, The Netherlands). Lock mass (used to correct each scan for mass error) was di-octylphtalate (DOP m/z 391.28429), this mass was present in the background in the whole chromatogram.

The described method was validated in order to test the following:
Within batch repeatability, between batch repeatability, sensitivity in standards, separation in standards, separation in biological samples (plasma extracts). Standards are not available for all the fatty acids and lipids, linearity was only determined for those that are available, see Table 2 A. For other fatty acids and lipids, a simple test was performed where 8, 10 and 12 microliters of the same plasma were extracted to see whether appropriate concentration changes would be detected. A summary of the results is shown in Table 2 B.

**Table 2 A.**

| Calibration ranges and linearity of the method for all available standards. | | | |
|---|---|---|---|
| Calibrations nM | high | low | r2 |
| C16:0* | 15653 | 16 | 0.991 |
| C16:0 SPM | 5715 | 6 | 0.999 |
| C16:1 * | 1546 | 2 | 0.994 |
| C18:0* | 13914 | 14 | 0.996 |
| C18:0 LPC | 7622 | 8 | 0.999 |
| C18:1 * | 14105 | 14 | 0.998 |
| C18:2* | 14256 | 14 | 0.999 |
| C18:3 w3* | 1074 | 1 | 0.999 |
| C18:3 w6* | 1078 | 1 | 0.999 |
| C20:3 w6* | 980 | 1 | 0.999 |
| C20:3 w9* | 980 | 1 | 0.999 |
| C20:4 w3* | 987 | 1 | 0.999 |
| C20:4 w6* | 984 | 1 | 0.999 |
| C22:5 w3* | 909 | 1 | 0.998 |
| C22:6 w3* | 915 | 1 | 0.999 |
| C34:0 PC | 516 | 1 | 0.985 |
| C36:2 PC | 5120 | 5 | 0.999 |

| | | | |
|---|---|---|---|
| * acidic group derivatised with GTMAC | | | |

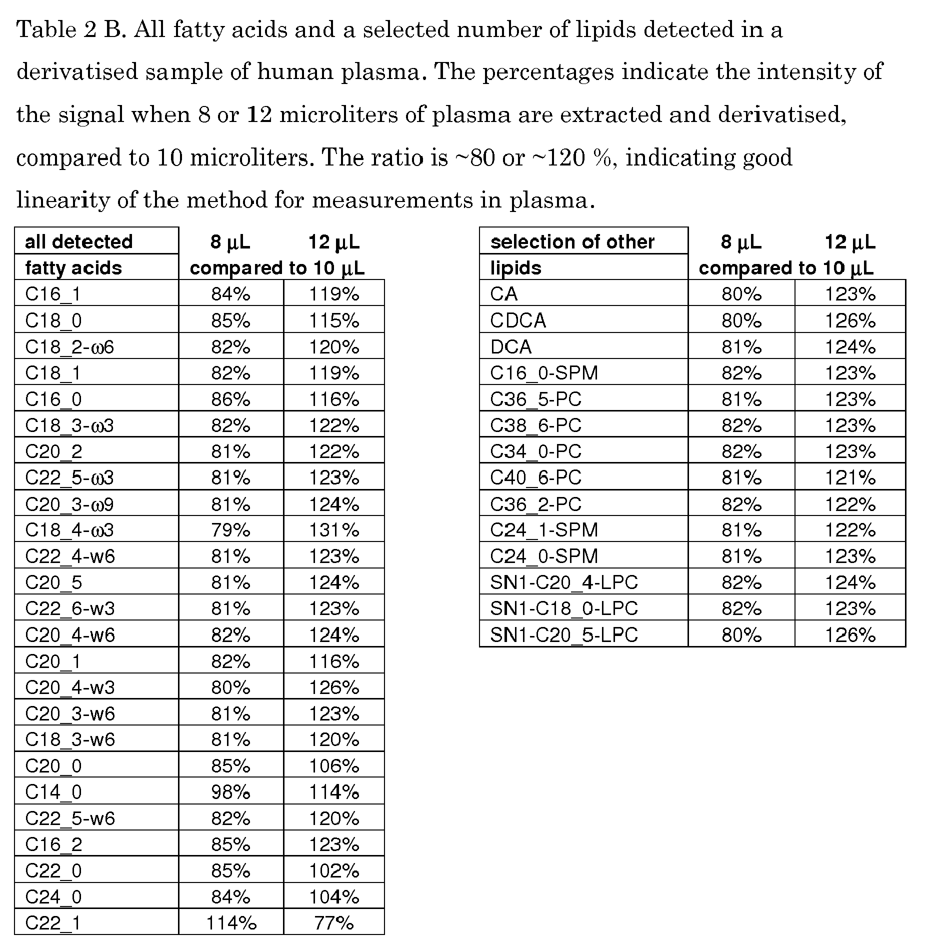

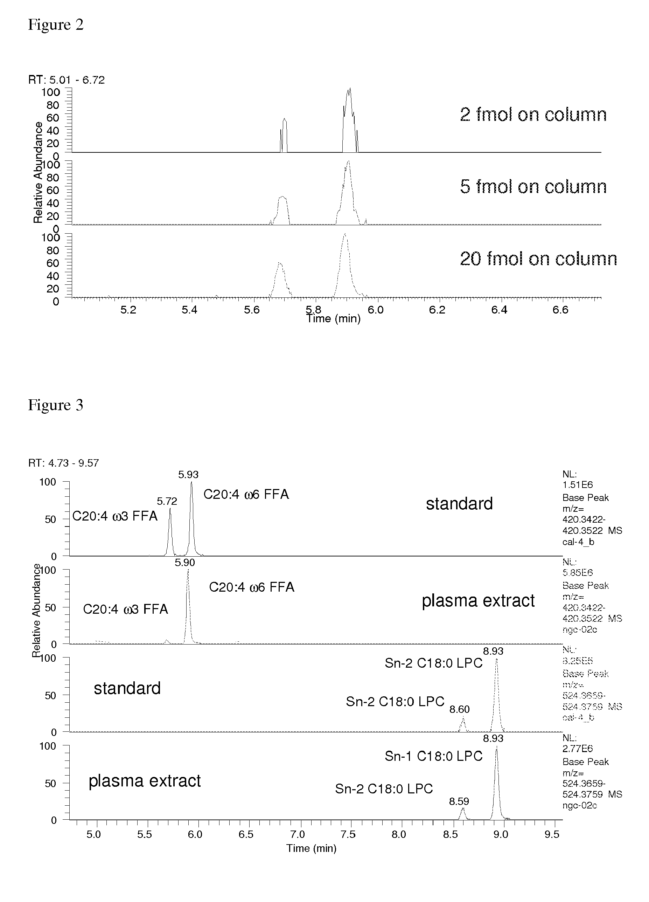

The method for derivatised free fatty acids is very sensitive, as can be seen in Figure 2. Amounts as low as 2 fmol injected onto the column can still easily be detected. This is very important especially when analysing conjugated unsaturated fatty acids.

Within batch precision was determined for 24 fatty acids detected in human plasma and varied from 3% to 15%. Between batch precision varied from 1% to 32 %. The latter was for 16:2 free fatty acid which is a fatty acid present in very low concentrations in plasma.

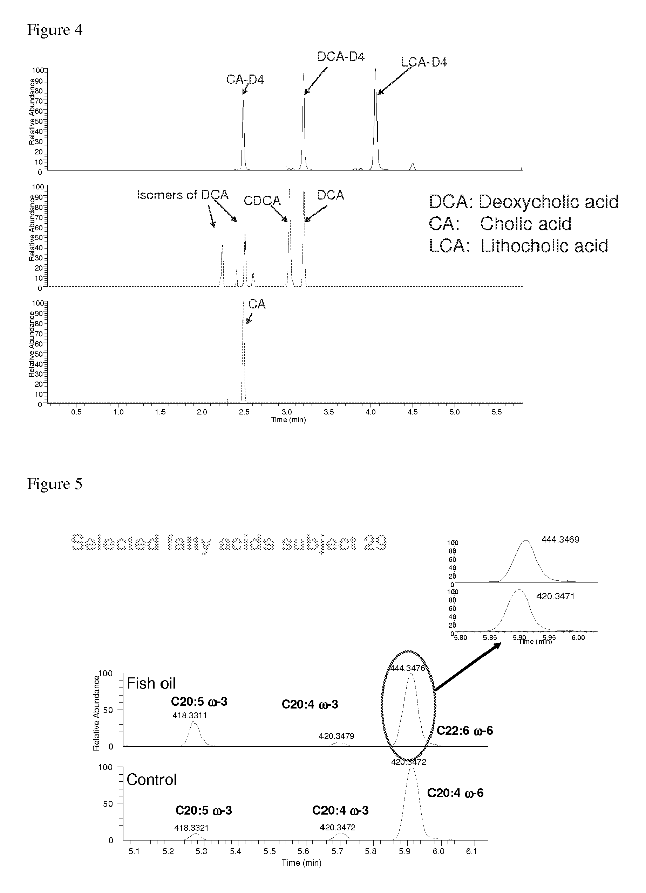

In addition to the 24 free fatty acids, in human plasma, about 20 lysophosphatidylcholines are detected, 12 sphingomyelines, and 20 phosphatidylcholines can be detected, as well as the most abundant bile acids in plasma, cholic acid and deoxycholic acid. This is shown in Figures 3 and 4.

In order to test the method in a relevant setting, plasma samples from a study with fish oil were analysed. In figure 5 an example is shown from plasma taken from the same subject after receiving two treatments: control treatment with vegetable oil and fish oil. In the figure it can be seen that fatty acids present in fish oil can be detected in human plasma after fish oil supplementation.

## Claims

1. Method to make a sample of fatty acids and bile acids suitable for analysis with liquid chromatography followed by mass spectrography (LC-MS) comprising derivatising said fatty acids and bile acids in the sample with glycidyltrimethylammonium chloride (GTMAC).

2. Method for analyzing lipids comprising:
a. subjecting a sample comprising lipids and free fatty or bile acids to derivatisation with GTMAC; and
b. performing LC-MS on said derivatised fatty or bile acids in the presence of intact lipids.

3. Method according to claim 1 or 2, wherein the sample comprises fatty acids, more particularly polyunsaturated fatty acids (PUFAs).

4. Method according to any of the previous claims, wherein said sample is a biological sample, more preferably a blood, plasma or serum sample.

5. Method according to any of the previous claims, wherein the LC is ultra-high pressure liquid chromatography (UHPLC).

6. Method according to one of the previous claims, wherein the MS analysis is performed by electrospray (ESI).

7. Method according to any of the previous claims, wherein the derivatisation is performed at a temperature between 0°C and 90°C, preferably between room temperature and 80°C, more preferably around 65°C.

8. Method according to any of the previous claims, wherein the derivatisation reaction is performed for more than 30 minutes, preferably for more than 60 minutes, more preferably for 85 minutes or more, and most preferably between about 87 and about 93 minutes.

9. Use of GTMAC for derivatisation of fatty and bile acids in an LC-MS analysis of a sample comprising said lipids.
